# EUROPÄISCHE PATENTSCHRIFT

(11) **EP 0 643 712 B1**
(45) Veröffentlichungstag und Bekanntmachung des Hinweises auf die Patenterteilung: **26.11.1997**
(21) Anmeldenummer: 93912829.4
(22) Anmeldetag: 29.05.1993
(51) Int. Cl.: C07D 493/10, C07D 303/32, C07D 307/94, A61K 31/335

(54) **SPIRO-OXETANE, VERFAHREN ZU IHRER HERSTELLUNG UND ARZNEIMITTEL**
SPIRO-OXETANES, PROCESS FOR PREPARING THE SAME AND MEDICAMENTS
SPIRO-OXETANES, LEUR PROCEDE DE PREPARATION ET MEDICAMENTS

(30) Priorität: 02.06.1992 DE 4218096
(43) Veröffentlichungstag der Anmeldung: 22.03.1995
(73) Patentinhaber: BOEHRINGER MANNHEIM GMBH, 68305 Mannheim (DE)
(72) Erfinder: FRIEBE, Walter-Gunar, D-6800 Mannheim 1 (DE); SCHEUER, Werner, D-8122 Penzberg (DE); TIBES, Ulrich, D-600 Frankfurt 70 (DE)
(86) Internationale Anmeldenummer: EP9301356
(87) Internationale Veröffentlichungsnummer: WO9324492

(56) Entgegenhaltungen:
- CH-A- 613 699
- LIEBIGS ANNALEN DER CHEMIE 1988, WEINHEIM, DE, Seiten 869-875, W. ADAM et al.

## Beschreibung

Gegenstand der vorliegenden Erfindung sind Arzneimittel, die Spiro-oxetanderivate enthalten, Verfahren zu deren Herstellung sowie neue Spiro-oxetanderivate.

Die Erfindung betrifft Arzneimittel, die Spiro-oxetanderivate der allgemeine Formel I enthalten 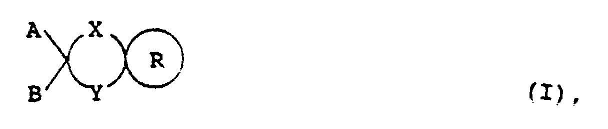 in welcher,
- A und B,: die gleich oder verschieden sind, jeweils einen gewünschtenfalls durch Halogen, Hydroxy, C₁-C₆-Alkyl oder C₁-C₆-Alkoxy substituierten Phenylrest oder gemeinsam eine Gruppe -CH=CH-CO-CH=CH-,
- X: ein Sauerstoff und Y eine Methylengruppe oder
- X: eine Gruppe -CH₂O- und Y eine Valenzbindung und
- R: einen spiroverknüpften C₃-C₆-Cycloalkanrest oder einen spiroverknüpften C₃-C₆-Lactonrest
bedeuten,
deren Enantiomere, Diastereomere und Racemate.

Die Verbindungen der allgemeinen Formel I weisen wertvolle pharmakologische Eigenschaften auf, insbesondere können sie die Aktivität von Phospholipasen hemmen. Sie eignen sich daher zur Behandlung akuter und chronischer allergischer, nichtallergischer und traumatischer entzündlicher Erkrankungen wie beispielsweise Rheumatische Arthritis, Osteoarthritis, Ulcerative Colitis, Akute Pankreatitis, Kontaktdermatitis und Entzündliche Atemwegserkrankungen.

Aus Liebigs Ann. Chem. 1988, 869 sind einige Spiro-oxetane bekannt, jedoch ohne Angabe einer pharmazeutischen Wirkung.

Gegenstand der Erfindung sind daher auch neue Spiro-oxetane der allgemeinen Formel I. 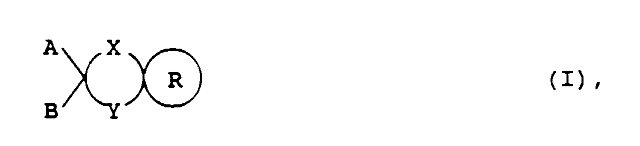 in welcher,
- A und B,: die gleich oder verschieden sind, jeweils einen gewünschtenfalls durch Halogen, Hydroxy, C₁-C₆-Alkyl oder C₁-C₆-Alkoxy substituierten Phenylrest oder gemeinsam eine Gruppe -CH=CH-CO-CH=CH-,
- X: ein Sauerstoff und Y eine Methylengruppe oder
- X: eine Gruppe -CH₂O- und Y eine Valenzbindung und
- R: einen spiroverknüpften C₃-C₆-Cycloalkanrest oder einen spiroverknüpften C₅- oder C₆-Lactonrest
bedeuten,
deren Enantiomere, Diastereomere und Racemate.

Ferner sind Gegenstand der Erfindung neue Spiro-oxetane der Formel I, in welcher
A und B, die gleich oder verschieden sind, jeweils einen durch Halogen, Hydroxy, C₁-C₆-Alkyl oder C₁-C₆-Alkoxy substituierten Phenylrest,
- X: ein Sauerstoff und Y eine Methylengruppe oder
- X: eine Gruppe -CH₂O- und Y eine Valenzbindung und
- R: einen spiroverknüpften C₃- oder C₄-Lactonrest bedeuten,
deren Enantiomere, Diastereomere und Racemate sowie die Verbindung 1,5-Dioxadispiro[3.1.5.1]dodeca-7,10-dien-9-on.

Die Alkylrest in den genannten Gruppen können geradkettig oder verzweigt sein. Bevorzugte Alkylreste sind der Methyl-, Ethyl-, n-Propyl-, Isopropyl-, n-Butyl-, Isobutyl-, tert.-Butyl-, n-Pentyl- und 3-Pentylrest.

Alkoxy bedeutet vorzugsweise Methoxy, Ethoxy, Propoxy oder Butoxy.

Halogenatome sind insbesondere Fluor, Chlor und Brom.

Cycloalkylreste bedeuten vorzugsweise Cyclopropyl, Cyclobutyl, Cyclopentyl oder Cyclohexyl.

Als Lactonrest kommen β-Propiolacton, γ-Butyrolacton, δ-Valerolacton und ε-Caprolacton in Frage.

Außer den in den Beispielen genannten Verbindungen sind Gegenstand der Erfindung insbesondere alle Substanzen, die jede mögliche Kombination der in den Beispielen genannten Substituenten aufweisen.

Das erfindungsgemäße Verfahren zur Herstellung der Verbindungen der Formel I ist dadurch gekennzeichnet, daß man in an sich bekannter Weise entweder
a) eine Verbindung der allgemeinen Formel II 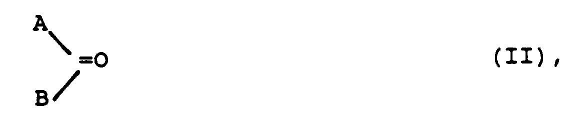 in welcher A und B die obengenannte Bedeutung haben, mit einer Verbindung der allgemeinen Formel III

   H₂C=® (III),

   in welcher ® die obengenannte Bedeutung hat, oder
b) eine Verbindung der allgemeinen Formel IV 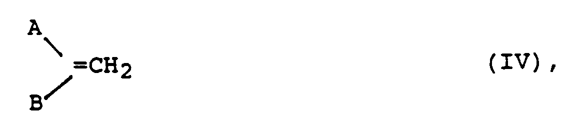 in welcher A und B die obengenannte Bedeutung haben, mit einer Verbindung der allgemeinen Formel V

   O=® (V),

   in welcher ® die obengenannte Bedeutung hat,
umsetzt, das erhaltene Reaktionsgemisch aufarbeitet, die Verbindungen der Formel I isoliert und anschließend gewünschtenfalls die erhaltenen Verbindungen I in ihre Enantiomeren oder Diasteromeren aufspaltet.

Die Umsetzung von Verbindungen der Formel II mit Verbindungen der Formel III oder Verbindungen der Formel IV mit Verbindungen der Formel V erfolgt zweckmäßig in einem Lösungsmittel wie beispielsweise einem Halogenkohlenwasserstoff wie Dichlormethan, Chloroform, Tetrachlormethan oder Trichlorethan unter Lichteinwirkung wie beispielsweise Sonnenlicht, Glühlampenlicht, UV-Licht oder UV/VIS-Laserlicht.

Die Trennung von Racematen in die Enantiomeren oder Diastereomeren kann chromatographisch auf geeigneten optisch aktiven Phasen mit gängigen Elutionsmitteln durchgeführt werden. Als optisch aktive Phasen eignen sich beispielsweise optisch aktive Polyacrylamide oder Polymethacrylamide, z. T. auch Kieselgel (z. B. ChiraSpher (R) von Merck, Chiralpak (R) OT/OP von Baker), Celluloseester/-Carbamate (z. B. Chiracel (R) OB/OY von Baker/Daicel), Phasen auf Cyclodextrin- oder Kronenetherbasis (z. B. Crownpak (R) von Daicel) oder mikrokristallines Cellulosetriacetat (Merck).

Die Ausgangsverbindungen II, III, IV und V sind literaturbekannte Substanzen oder können in Analogie zu literaturbekannten Verfahren hergestellt werden.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und / oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Gemschmacks- und Süßstoffe enthalten. Für die äußerliche Anwendung können die erfindungsgemäßen Substanzen I auch in Form von Pudern und Salben verwendet werden. Sie werden dazu z.B. mit pulverförmigen, physiologisch verträglichen Verdünnungsmitteln bzw. üblichen Salbengrundlagen vermischt.

Die verabreichte Dosis hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Außer den in den Beispielen genannten Substanzen sind im Sinne der vorliegenden Erfindung die folgenden Verbindungen bevorzugt:
6-Oxadispiro[4.1.5.1]trideca-8,11-dien-10-on
2,6-Dioxadispiro[4.1.5.1]trideca-8,11-dien-1,10-dion
2,2-Di-(4-methoxy-phenyl)-1,5-dioxaspiro[3.4]octan-6-on
1,1-Di-(4-methoxy-phenyl)-2,5-dioxaspiro[3.4]octan-6-on
2,2-Di-(4-methyl-phenyl)-1,5-dioxaspiro[3.4]octan-6-on
1,1-Di-(4-methyl-phenyl)-2,5-dioxaspiro[3.4]octan-6-on
2,2-Di-(4-chlor-phenyl)-1,5-dioxaspiro[3.4]octan-6-on
1,1-Di-(4-chlor-phenyl)-2,5-dioxaspiro[3.4]octan-6-on

### Beispiel 1

### 1,6-Dioxadispiro[4.1.5.1]trideca-8,11-dien-2,10-dion

Eine Lösung von 6 ml (66 mmol)γ-Methylen-γ-butyrolacton und 7.2 g (66 mmol) p-Benzochinon in 400 ml Tetrachlormethan wird bei 0 bis 5°C 7 Stunden mit UV-Licht bestrahlt. Man filtriert den Niederschlag ab und reinigt an Kieselgel (Elutionsmittel Isohexan:Ethylacetat 3:1). Nach Verreiben mit Ether verbleiben 2.2 g Titelverbindung (16 % d. Th.) vom Schmp. 156 - 158°C.

### Beispiel 2

In analoger Weise wie in Beispiel 1 beschrieben erhält man aus p-Benzochinon und der jeweiligen Methylenverbindung:

| Bezeichnung | | Ausb. % | Schmelzpunkt °C (Lsg.mittel) |
|---|---|---|---|
| a) | 4-Oxadispiro[2.1.5.1]undeca-6,9-dien-8-on (Fraktion 1) sowie | 11 | Öl |
| b) | 10-Oxadispiro[2.0.5.2]undeca-5,8-dien-7-on (Fraktion 2) aus Methylencyclopropan | 9 | Öl |
| c) | 5-Oxadispiro[3.1.5.1]dodeca-7,10-dien-9-on aus Methylencyclobutan | 8 | Öl |
| d) | 1,5-Dioxadispiro[3.1.5.1]dodeca-7,10-dien-2,9-dion aus Diketen | 12 | 70-72 |

### Beispiel 3

In analoger Weise wie in Beispiel 1 beschrieben erhält man aus Benzophenon und γ-Methylen-γ-butyrolacton in 11 % Ausbeute 2,2-Diphenyl-1,5-dioxaspiro[3.4]octan-6-on vom Schmp. 130 - 132°C sowie in 14 % Ausbeute 1,1-Diphenyl-2,5-dioxaspiro[3.4]octan-6-on vom Schmp. 138 - 140°C.

### Pharmakologische Tests

### Wirkungen der Spiro-Oxetane

Die vorliegende Anmeldung betrifft Arzneimittel zur Behandlung akuter und chronischer Erkrankungen mit entzündlicher, immunologischer, allergischer, nicht-allergischer oder traumatischer Genese.

Da die Phospholipase A₂ (PLA₂) ein Schlüsselenzym bei der Genese dieser Erkrankungen ist, werden durch ihre Inhibition diese Erkrankungen wirkungsvoll bekämpft.

Die Spiro-Oxetane sind nun PLA₂-Hemmer, die in ihrem Wirkprofil den eingeführten und therapeutisch bewährten Cyclooxygenase-inhibitoren überlegen sind. Zum Vergleich wurde mit Indomethacin ein typischer CO-Inhibitor ausgewählt.

### 1) Hemmuna der PLA₂

Als typischer Vertreter einer PLA₂ wurde die humane rekombinante Typ-II-PLA₂ (= synoviale PLA₂) zur Testung eingesetzt.

Die Tabelle 1 enthält die prozentuale in-vitro Hemmung dieses Enzyms durch die Verbindung des Beispiels 1, einen Spiro-Oxetan-Vertreter. Das Enzym wird dosisabhängig bis zu 93 % inhibiert.

Demgegenüber sieht man in Tabelle 1, daß Indomethacin das Enzym zwar auch hemmt, jedoch nur bei maximal 52 % bei der höchsten Konzentration. Hiermit dokumentiert sich bereits eine Überlegenheit.

### 2) Hemmung der collageninduzierten AA-Freisetzung aus humanen Thrombozyten

Als weiterer Indikator einer PLA₂-Inhibition gilt eine Hemmung der AA-Freisetzung aus Thrombozyten. Zu diesem Zweck werden die Thrombozyten mit ³H-AA inkubiert. Diese zugegebene radioaktive Arachidonsäure baut sich in Membranphospholipide ein. Anschließend wird die thrombozytäre PLA₂ über den Collagenrezeptor aktiviert und ³H-AA so aus Phospholipen wieder ins Medium freigesetzt, wo sie quantifiziert werden kann.

In Tabelle 1 ist gezeigt, daß mit der Verbindung des Beispiels 1 die collageninduzierte AA-Freisetzung dosisabhängig gehemmt wurde, wobei mit 100 % die Hemmung bei der höchsten Konzentration des Inhibitors vollständig war. Demgegenüber zeigt Indomethacin bei der höchsten Konzentration nur eine 23%ige Hemmung. .

Die geringere Hemmung sowohl der rh sPLA₂ als auch der AA-Freisetzung durch Indomethacin veranschaulichen bereits, daß diese Struktur keine wesentliche PLA₂ inhibitierende Wirkung besitzt. Demgegenüber hemmte der Spiro-Oxetan-Vertreter das Enzym und die AA-Freisetzung vollständig und ist hierdurch überlegen.

**Tab. 1**

| Vergleich des Wirkungsspektrums von der Verbindung des Beispiels 1 und Indomethacin | | |
|---|---|---|
| **Substanz** | **PLA**_{**2**} ^{**a)**} in % | **AA** ^{**b)**} in % |
| **Bsp. 1** | | |
| **100 µg/ml** | 93 | 100 |
| **10 µg/ml** | 37 | 86 |
| **1 µg/ml** | 11 | 42 |

| **Indomethacin** | | |
|---|---|---|
| **100 µg/ml** | 52 | 23 |
| **10 µg/ml** | 10 | 18 |
| **1 µg/ml** | 0 | 6 |

| | | |
|---|---|---|
| ^{a)} Hemmung der Enzymaktivität (humane rekombinante Typ II PLA₂) | | |
| ^{b)} Hemmung der Collagen induzierten Arachidonsäurefreisetzung | | |

## Patentansprüche

1. Arzneimittel, enthaltend neben üblichen Träger- und Hilfsstoffen eine Verbindung der Formel I 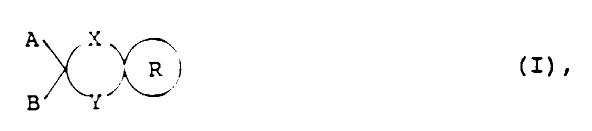 in welcher,
A und B, die gleich oder verschieden sind, jeweils einen gewünschtenfalls durch Halogen, Hydroxy, C₁-C₆-Alkyl oder C₁-C₆-Alkoxy substituierten Phenylrest oder gemeinsam eine Gruppe -CH=CH-CO-CH=CH-,
X ein Sauerstoff und Y eine Methylengruppe oder
X eine Gruppe -CH₂O- und Y eine Valenzbindung und
R einen spiroverknüpften C₃-C₆-Cycloalkanrest oder einen spiroverknüpften C₃-C₆-Lactonrest
bedeuten,
deren Enantiomere, Diastereomere und Racemate.

2. Verwendung von Verbindungen der Formel I gemäß Anspruch 1 zur Herstellung von Arzneimitteln zur Behandlung akuter und chronischer allergischer, nicht-allgergischer und traumatischer entzündlicher Erkrankungen.

3. Verbindungen der Formel I 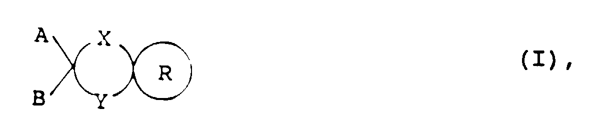 in welcher,
A und B, die gleich oder verschieden sind, jeweils einen gewünschtenfalls durch Halogen, Hydroxy, C₁-C₆-Alkyl oder C₁-C₆-Alkoxy substituierten Phenylrest oder gemeinsam eine Gruppe -CH=CH-CO-CH=CH-,
X ein Sauerstoff und Y eine Methylengruppe oder
X eine Gruppe -CH₂O- und Y eine Valenzbindung und
R einen spiroverknüpften C₃-C₆-Cycloalkanrest oder einen spiroverknüpften C₅- oder C₆-Lactonrest
bedeuten,
deren Enantiomere, Diastereomere und Racemate.

4. Verbindungen der Formel I 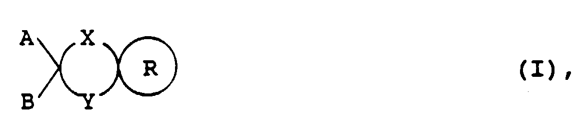 in welcher,
A und B, die gleich oder verschieden sind, jeweils einen durch Halogen, Hydroxy, C₁-C₆-Alkyl oder C₁-C₆-Alkoxy substituierten Phenylrest,
X ein Sauerstoff und Y eine Methylengruppe oder
X eine Gruppe -CH₂O- und Y eine Valenzbindung und
R einen spiroverknüpften C₃- oder C₄-Lactonrest
bedeuten,
deren Enantiomere, Diastereomere und Racemate.

5. 5-Dioxadispiro[3.1.5.1]-dodeca-7,10-dien-2,9-dion.

6. Verfahren zur Herstellung von Verbindungen der Formel I gemäß einem der Ansprüche 3 bis 5,
dadurch gekennzeichnet, daß man in an sich bekannter Weise entweder
a) eine Verbindung der allgemeinen Formel II 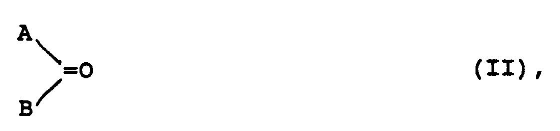 in welcher A und B die obengenannte Bedeutung haben,
mit einer Verbindung der allgemeinen Formel III
H₂C=® (III),
in welcher ® die obengenannte Bedeutung hat, oder
b) eine Verbindung der allgemeinen Formel IV 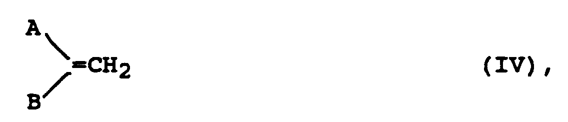 in welcher A und B die obengenannte Bedeutung haben,
mit einer Verbindung der allgemeinen Formel V
O=® (V),
in welcher® die obengenannte Bedeutung hat,
umsetzt, das erhaltene Reaktionsgemisch aufarbeitet, die Verbindungen der Formel I isoliert und anschließend gewünschtenfalls die erhaltenen Verbindungen I in ihre Enantiomeren oder Diasteromeren aufspaltet.

## Claims

1. Pharmaceutical agents containing a compound of formula I in addition to conventional carrier and auxiliary substances 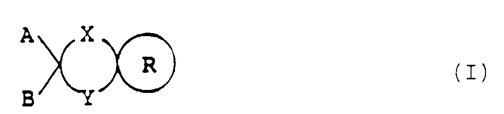 in which
A and B which are the same or different, each denote a phenyl residue which is substituted if desired, by halogen, hydroxy, C₁-C₆ alkyl or C₁-C₆ alkoxy or together denote the group -CH=CH-CO-CH=CH-,
X denotes oxygen and Y denotes a methylene group or
X denotes the group -CH₂O- and Y is a valency bond and
R denotes a spiro-linked C₃-C₆ cycloalkane residue or a spiro-linked C₃-C₆ lactone residue,
their enantiomers, diastereomers and racemates.

2. Use of compounds of formula I as claimed in claim 1 for the treatment of acute and chronic allergic, non-allergic and traumatic inflammatory diseases.

3. Compounds of formula I 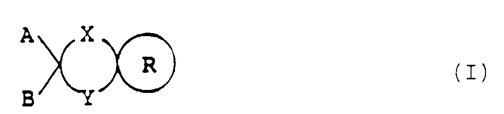 in which
A and B which are the same or different, each denote a phenyl residue which is substituted if desired, by halogen, hydroxy, C₁-C₆ alkyl or C₁-C₆ alkoxy or together denote the group -CH=CH-CO-CH=CH-,
X denotes oxygen and Y denotes a methylene group or
X denotes the group -CH₂O- and Y is a valency bond and
R denotes a spiro-linked C₃-C₆ cycloalkane residue or a spiro-linked C₅ or C₆ lactone residue,
their enantiomers, diastereomers and racemates.

4. Compounds of formula I 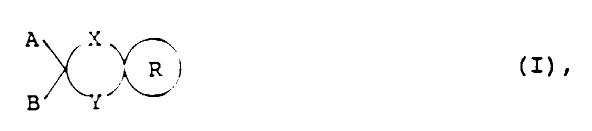 in which
A and B which are the same or different, each denote a phenyl residue which is substituted if desired, by halogen, hydroxy, C₁-C₆ alkyl or C₁-C₆ alkoxy,
x denotes oxygen and Y denotes a methylene group or
x denotes the group -CH₂O- and Y is a valency bond and
R denotes a spiro-linked C₃ or C₄ lactone residue,
their enantiomers, diastereomers and racemates.

5. 5-dioxadispiro[3.1.5.1]-dodeca-7,10-dien-2,9-dione.

6. Process for the production of compounds of formula I as claimed in one of the claims 3 to 5,
wherein either
a) a compound of the general formula II 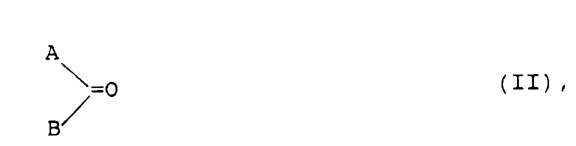 in which A and B have the above-mentioned meaning
is reacted in a well-known manner with a compound of the general formula III
H₂C=® (III)
in which ® has the meaning stated above or
b) a compound of the general formula IV 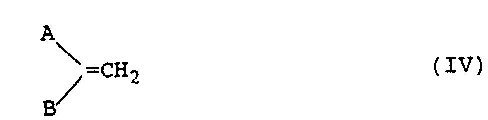 in which A and B have the meaning stated above
is reacted in a well-known manner with a compound of the general formula V
O=® (V)
in which ® has the meaning stated above,
the reaction mixture obtained is processed, the compounds of formula I are isolated and subsequently if desired the compounds I obtained are resolved into their enantiomers or diastereomers.

## Revendications

1. Médicament, contenant, outre les véhicules et les excipients habituels, un composé de formule I : 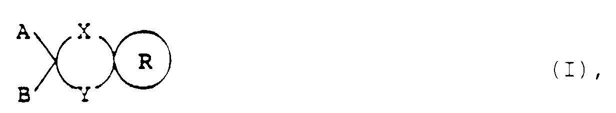 dans laquelle,
A et B, qui sont identiques ou différents, représentent chacun un résidu phénylique substitué à souhait par un radical halogéno, hydroxy, alkyl en C₁ à C₆ ou alcoxy en C₁ à C₆ ou ensemble un groupement -CH=CH-CO-CH=CH-,
X un atome d'oxygène et Y un groupement méthylène ou bien
X un groupement -CH₂O- et Y une liaison de valence et
R un résidu cycloalcane en C₃ à C₆ à liaison spiro ou bien un résidu lactone en C₃ à C₆ à liaison spiro, leurs énantiomères, diastéréomères et racémates.

2. Utilisation de composés de formule I selon la revendication 1 pour la fabrication de médicaments traitant les maladies inflammatoires, traumatiques, allergiques aiguës et chroniques, et non-allergiques.

3. Composés de formule I : 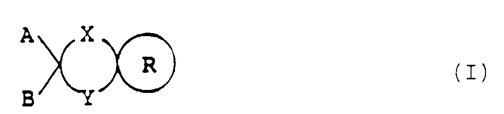 dans laquelle,
A et B, qui sont identiques ou différents, représentent chacun un résidu phénylique substitué à souhait par un radical halogéno, hydroxy, alkyl en C₁ à C₆ ou alcoxy en C₁ à C₆ ou ensemble un groupement -CH=CH-CO-CH=CH-,
X un atome d'oxygène et Y un groupement méthylène ou bien
X un groupement -CH₂O- et Y une liaison de valence et
R un résidu cycloalcane en C₃ à C₆ à liaison spiro ou bien un résidu lactone en C₃ à C₆ à liaison spiro, leurs énantiomères, diastéréomères et racémates.

4. Composés de formule I : 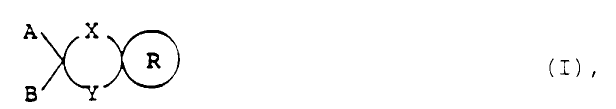 dans laquelle,
A et B, qui sont identiques ou différents, représentent chacun un résidu phénylique substitué à souhait par un radical halogéno, hydroxy, alkyl en C₁ à C₆ ou alcoxy en C₁ à C₆ ou ensemble un groupement -CH=CH-CO-CH=CH-,
X un atome d'oxygène et Y un groupement méthylène ou bien
X un groupement -CH₂O- et Y une liaison de valence et
R un résidu lactone en C₃ ou C₄ à liaison spiro, leurs énantiomères, diastéréomères et racémates.

5. La 5-dioxadispiro[3.1.5.1]-dodéca-7,10-diène-2,9-dione.

6. Procédé de fabrication de composés de formule I selon l'une quelconque des revendications 3 à 5, caractérisé en ce que l'on met en oeuvre d'une façon connue en elle-même soit
a) un composé de formule générale II 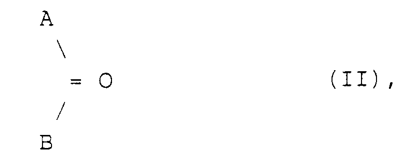 dans laquelle A et B ont la signification précitée, avec un composé de formule générale III
H₂C=® (III),
dans laquelle ® a la signification précitée, soit
b) un composé de formule générale IV 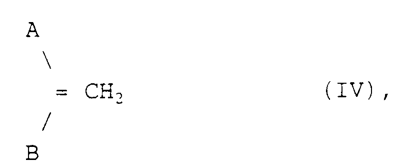 dans laquelle A et B ont la signification précitée, avec un composé de formule générale V
O=® (V),
dans laquelle ® a la signification précitée,
on achève le mélange réactionnel, on isole les composés de formule I, et finalement on sépare les composés obtenus I en leurs énantiomères ou diastéréomères.
